# EUROPÄISCHE PATENTSCHRIFT

(11) **EP 1 541 239 B1**
(45) Veröffentlichungstag und Bekanntmachung des Hinweises auf die Patenterteilung: **11.05.2011**
(21) Anmeldenummer: 04025610.9
(22) Anmeldetag: 28.10.2004
(51) Int. Cl.: B02C 19/22, B65G 65/32

(54) **Vorrichtung zum Zerreissen von Feststoffen, Zumischen von Flüssigkeit und Fördern der erzeugten Suspension in eine Verarbeitungsanlage**
Device for shredding of solids, adding of liquid and transporting of made suspension into converting plant
Dispositif pour déchirer de matière solide, ajouter du fluide et transporter de la suspension à une installation

(30) Priorität: 07.11.2003 DE 20317305 U
(43) Veröffentlichungstag der Anmeldung: 15.06.2005
(73) Patentinhaber: Hugo Vogelsang Maschinenbau GmbH, 49632 Essen (DE)
(72) Erfinder: Krampe, Paul, 49632 Essen/ Oldenburg (DE)
(74) Vertreter: Eisenführ, Speiser & Partner

(56) Entgegenhaltungen:
- DE-A1- 4 235 528
- DE-A1- 19 628 983
- DE-U1- 8 319 047
- US-A- 6 050 456

## Beschreibung

Die Erfindung bezieht sich auf eine Vorrichtung zum Zerreißen von Feststoffen, Zumischen von Flüssigkeit und Fördern der erzeugten Suspension in eine Verarbeitungsanlage; insbesondere von Biosubstrat-Gülle-Suspension in eine Biogasanlage, mit Zuführeinrichtungen für die Feststoffe und die Flüssigkeit, einer Schneckenanordnung und einer Pumpeinrichtung zum Austragen der Suspension.

Am häufigsten auf dem hier in Frage kommenden Sektor, nämlich bei der Förderung einer Biosubstrat-Gülle-Suspension in eine Biogasanlage, werden Schneckenanordnungen eingesetzt, deren Schnecken nur einseitig gelagert sind und an dem dem Lager gegenüberliegenden Ende lediglich ein Stützlager aufweisen. Dies führt häufig zur Berührung der Schnecke mit dem umgebenden Gehäuse und damit zu Verschleiß, was nur dann akzeptiert werden kann, wenn die Schnecke sehr langsam angetrieben wird, um Reibung im Gehäuse zu vermeiden.

Aus DE 83 19 047 U1 ist eine Vorrichtung zum dosierten Befeuchten und Weiterfördern von schwer pumpbaren Futtermitteln bekannt. Die Vorrichtung umfasst eine Mühle zum Zerkleinem von Futtermittel und eine Ringleitung, aus der das Futtermittel in einem Einlauftrichter einer Förderschnecke befeuchtet werden kann. Das solcherart befeuchtete und durch die Schnecke geförderte Futtermittel wird über eine Drehkolbenpumpe weitergepumpt.

Aus DE 196 28 983 A1 ist eine Kombination einer Rotationspumpe mit einem Fremdkörperzerkleinerer bekannt. Der Fremdkörperzerkleinerer besteht aus mehreren koaxial zueinander angeordneten Klingen, die mit in einem Gehäuse befestigten Gegenschneiden zusammenwirken. Die Rotationspumpe ist als Drehkolbenpumpe ausgeführt. Die Antriebswelle der Rotationspumpe liegt koaxial zur Welle des Fremdkörperzerkleinerers.

Aus US 6,050,456 ist eine Fördervorrichtung mit einer Doppelschneckenanordnung mit gegenläufigen Schnecken bekannt.

Aus DE 42 35 528 A1 ist eine Vorrichtung zum Dosieren und Mischen von zwei unterschiedlichen Viskosemassen bekannt. Die Vorrichtung umfasst zwei baugleiche, nebeneinander angeordnete Einheiten, die jeweils eine Drehkolbenpumpe und eine Mischeinrichtung umfassen.

Ausgangspunkt der vorliegenden Erfindung ist eine Vorrichtung der eingangs genannten Art, und es geht darum, diese bekannten Vorrichtungen so zu verbessern, dass die nachfolgend genannten Merkmale, Eigenheiten und Vorteile erreicht werden können.

Die Vorrichtung gemäß der Erfindung ist ausgehend von dem einleitend genannten Oberbegriff gekennzeichnet durch eine Doppelschneckenanordnung mit zwei parallel nebeneinander angeordneten angetriebenen Wellen, auf denen jeweils zusammenwirkend je ein Förder-, Zerreiß- und Mischschneckenteil und je ein Drehkolben einer gemeinsamen Drehkolbenpumpe angeordnet sind, und durch Lagerungen der Wellen bzw. Schnecken jeweils an beiden Enden.

Bei weiteren Ausführungsformen der Erfindung steht für beide Wellen ein einziger gemeinsamer Antrieb zur Verfügung, der, wenn es erforderlich sein sollte, auch reversiert betrieben werden kann.

Bei bevorzugten Ausführungsformen der Erfindung wird die Flüssigkeit in den vorderen Bereich der Schnecken, d.h. in den Zerreiß- und Mischbereich, eingeleitet, und der Auslauf ist an der Drehkolbenpumpe nach oben hin gerichtet und unterhalb der Pumpe ist ein Steinabscheider vorgesehen.

Für beide Schnecken und die Pumpe ist vorzugsweise lediglich ein einziger Antrieb erforderlich, wobei die Schnecken gegenläufig arbeiten und genauso wie die Drehkolben zueinander passend ausgebildet sind.

Die Schnecken werden von oben befüllt, d.h. die Feststoffe fallen von oben nach unten, wohingegen die Drehkolbenpumpe am Ausgang der Vorrichtung von unten nach oben fördert, so dass sich kein Luftsack im Gehäuse bilden kann. Entsprechend ist dann auch nur ein einziges Synchronisiergetriebe sowohl für die Schnecken als auch für die Pumpe erforderlich.

Die Lagerung der beiden Wellen ist so an beiden Enden vorgesehen, dass die Lager von außen her gut erreicht werden können. Durch diese beidseitige Lagerung kann erreicht werden, dass die Schnecken das Gehäuse nicht berühren, so dass die Vorrichtung verschleißarm arbeitet und bei relativ hohen Drehzahlen eingesetzt werden kann, ohne dass es zu unzulässigen Temperaturerhöhungen kommen kann.

Durch den gegensinnigen Antrieb der Schnecken kann insbesondere das gefürchtete Aufwickeln von Faserstoffen verhindert werden. Andererseits ist möglich, den Antrieb kurzzeitig zu reversieren, um irgendwelche Fremdstoffe und Grobstoffe wieder zu lösen. Es kann also dadurch ein Reinigungs- oder sogar ein Selbstreinigungseffekt erzielt werden.

Die Schnecken sind so ausgebildet worden, dass am Außenrand der Gänge Zähne vorhanden sind, die eine Zerkleinerungsfunktion an den Feststoffen durchführen.

Insgesamt ist die Pumpenkammer und das gesamte Gehäuse der Vorrichtung gemäß der Erfindung von außen her leicht zugänglich, so dass leichte Demontagemöglichkeiten gegeben sind, wenn dies erforderlich sein sollte.

Weiterhin ist es möglich, den Trockensubstanzgehalt der Mischung durch die Menge der zugeführten Flüssigkeit genau einzustellen. Bei mit Flüssigkeit gefülltem Zuführtrichter schwimmen die Trockenstoffe auf der Flüssigkeit, d.h. wenig Trockenmaterial wird eingezogen. Durch Reduzierung der Flüssigkeitsmenge wird die von den Schnecken eingezogene Trockenstoffmenge dann entsprechend erhöht.

Mit Hilfe der Vorrichtung gemäß der Erfindung können auch hochviskose Suspensionen bei geringem Pumpenverschleiß gefördert werden. Die Pumpe leidet auch nicht unter Ansaugproblemen, da die Schnecken die Suspension in den Pumpbereich hineindrücken.

Mit Hilfe der Vorrichtung gemäß der Erfindung können beispielsweise mehrere Biogasanlagen oder Fermenter beschickt werden, indem die entsprechenden Rohrleitungsventile umgeschaltet werden.

Auch kann die Vorrichtung gemäß der Erfindung nach außen hin luftdicht abgeschlossen werden, so dass keine Geruchsbelästigung entstehen kann. Durch die Ausgestaltung der Schnecken, insbesondere auch durch die Zähne im Zerreiß- und Mischbereich, können die Feststoffe besser aufgeschlossen werden, und es ist eine höhere Durchmischung und damit eine höhere Gasausbeute in einer Biogasanlage möglich. Außerdem werden die Feststoffe, bevor sie dem Fermenter zugeführt werden, gut durchmischt, so dass sich keine Schwimmdecke bilden kann und zugleich die Rührleistung im Fermenter herabgesetzt werden kann.

Die Erfindung wird nachstehend anhand der Zeichnung beispielsweise erläutert.
- Fig. 1: zeigt eine Anordnung der Vorrichtung gemäß der Erfindung in einer Biogasanlage und
- Fig. 2: zeigt eine schaubildliche Ansicht der Vorrichtung gemäß der Erfindung, von der Teile zur besseren Darstellung entfernt wurden.

Das Besondere einer bevorzugten Vorrichtung nach der Erfindung ist die Kombination einer an sich bekannten Doppelschnecke mit einer an sich bekannten Drehkolbenpumpe in einem einzigen Aggregat und mit einem einzigen Antrieb, wobei die Flüssigkeitszufuhr so geregelt wird, dass das Mischungsverhältnis zwischen Trockenstoffen und Flüssigkeit in gewünschter Weise einstellbar ist.

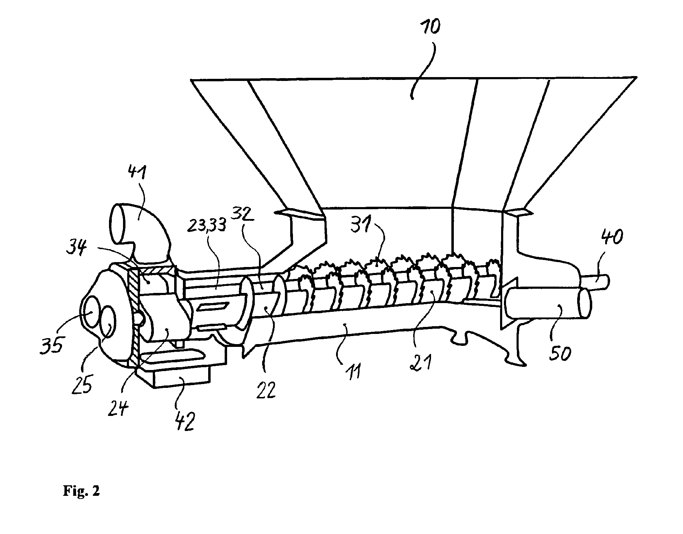

**Die in der** **Fig. 2** gezeigte Vorrichtung gemäß der Erfindung weist einen nach oben offenen Zuführtrichter 10 für die Feststoffe auf. Von daher gelangen die Feststoffe in den Innenraum eines Gehäuses 11, in welchem in Längsrichtung zwei zueinander parallele und im geringen Abstand zueinander angeordnete Wellen gelagert sind. Die Wellen sind im rechts gezeigten Bereich, im Zufuhrbereich, jeweils mit Zerreiß- und Mischschneckenteilen 21, 22 und 31, 32 ausgestattet. Es ist dieser Bereich, in dem die Feststoffe mechanisch bearbeitet werden, bevor sie durch die anschließenden Mischschneckenbereiche 22 und 32 weiterbefördert werden. Auf den Wellen befinden sich sodann zusammenwirkende Auswerfer und schließlich zwei zusammenwirkende Drehkolben 24 und 34 einer Drehkolbenpumpe. Linksseitig sind die beiden Wellen in den Lagern 25 und 35 gelagert. Entsprechende Lager befindet sich an der rechten Seite, jedoch sind diese Lager nicht gezeigt. Lediglich rechtsseitig ist ein Antrieb über einen Wellenstutzen 40 gezeigt, der über ein ebenfalls nicht gezeigtes Getriebe die beiden Schnecken bzw. Wellen gegenläufig dreht.

Oberhalb der Drehkolben 24 und 34 ist ein Auslass 41 gezeigt, während sich unterhalb der Kolben 24, 34 ein Steinabscheider 42 befindet.

Die zuzumischende Flüssigkeit wird über den Zulauf 50 in das Gehäuse 11 hineingefördert.

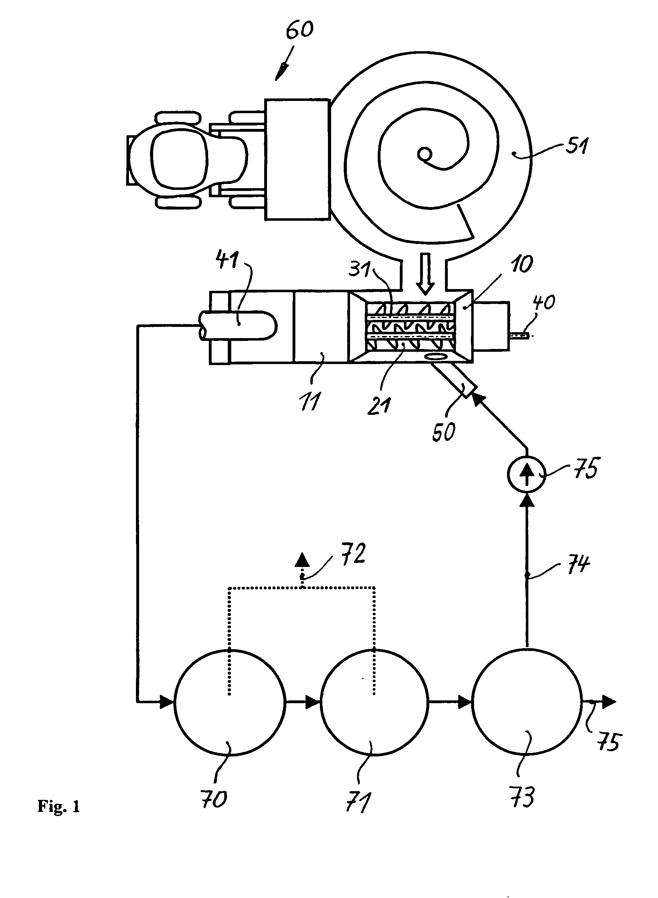

Fig. 1 zeigt die Vorrichtung gemäß der Erfindung in einer Anordnung für die Beschickung eines Fermenters einer Biogasanlage.

In der Fig. 1 sind das Gehäuse 11 und lediglich die wesentlichen Teile dieser Vorrichtung gezeigt, also der Zuführtrichter 10, der Anfangsbereich der beiden Schnecken 21 und 31, der Antrieb 40 sowie der Auslass 41. Mit 51 ist ein Feststoffmischer bezeichnet, der auf einem Frontladerfahrzeug 60 montiert ist und den Trichter 10 mit Feststoffen beschickt.

Die Flüssigkeit, die in das Gehäuse 11 hineingepumpt wird, wird über eine Pumpe 75 aus einem Endlager 73 entnommen. Das Endlager erhält die Flüssigkeit über zwei Fermenter 70 und 71, die bei 72 eine Biogasentnahme zeigen. Eine Gülleentnahme ist bei 75 gezeigt.

Die in der Fig. 1 gezeigte Vorrichtung dient einerseits zur Herstellung einer pumpfähigen Suspension aus trockenen Fermenten und Gülle mit einem definierten TS-Gehalt. Die Suspension wird sodann den Biogasfermentern 70 und 71 dosiert zugegeben.

Der Feststoffmischer 51 ist dann nicht erforderlich, wenn die Feststoffe auf anderem Wege, beispielsweise über ein Förderband dosiert zugeführt werden können. In diese Fall ist dann auch kein Frontlader oder dgl. erforderlich.

## Patentansprüche

1. Vorrichtung zum Zerreißen von Feststoffen, Zumischen von Flüssigkeit und Fördern der erzeugten Suspension in eine Verarbeitungsanlage; insbesondere von Biosubstrat-Gülle-Suspension in eine Biogasanlage, mit Zuführeinrichtungen für die Feststoffe (10) und die Flüssigkeit (50) einer Schneckenanordnung (21, 22, 31, 32) und einer Pumpeinrichtung (24, 34) zum Austragen der Suspension, **dadurch gekennzeichnet, dass** die Schneckenanordnung eine Doppelschneckenanordnung (21, 22, 31, 32) mit zwei parallelen nebeneinander angeordneten angetriebenen Wellen ist, auf denen jeweils zusammenwirkend je ein Förder-, Zerreiß- und Mischschneckenteil und je ein Drehkolben (24, 34) einer Drehkolbenpumpe angeordnet ist und dass Lagerungen der Wellen und Schnecken an beiden Enden (25, 35) bereitgestellt sind.

2. Vorrichtung nach Anspruch 1, **gekennzeichnet durch** einen gemeinsamen synchronisierten Antrieb (40) für beide Wellen (21, 22, 31, 32)

3. Vorrichtung nach Anspruch 2, **gekennzeichnet durch** einen reversierenden Antrieb (40).

4. Vorrichtung nach mindestens einem der Ansprüche 1 bis 3, **dadurch gekennzeichnet, dass** im Zerreiß- und Misch-Schneckenteil (21, 31) ein nach oben offener Zuführtrichter (10) als Zuführeinrichtung für Feststoffe angeordnet ist.

5. Vorrichtung nach mindestens einem der Ansprüche 1 bis 4, **dadurch gekennzeichnet, dass** die Zufuhr von Flüssigkeit (50) im Zerreiß- und Mischschneckenteil (21, 31) vorgesehen ist.

6. Vorrichtung nach mindestens einem der Ansprüche 1 bis 5, **dadurch gekennzeichnet, dass** die Drehkolbenpumpe (24, 34) einen nach oben gerichteten Auslass aufweist.

7. Vorrichtung nach Anspruch 6, **dadurch gekennzeichnet, dass** die Drehkolbenpumpe unterhalb des Pumpbereichs (24, 34) einen Steinabscheider (42) aufweist.

## Claims

1. Device for shredding of solids, adding of liquid and transporting of made suspension into converting plant; in particular for transporting a suspension of biosubstrate and liquid manure into a biogas plant, with feed means for the solids (10) and the liquid (50) a screw arrangement (21, 22, 31, 32) and a pumping means (24, 34) for discharging the suspension, **characterized in that** the screw arrangement is a twin screw arrangement (21, 22, 31, 32) having two driven shafts which are arranged parallel next to one another and on each of which a respective conveying, shredding and mixing screw part and a respective rotary piston (24, 34) of a rotary piston pump are arranged such that they cooperate, and **in that** bearings are provided for the shafts and screws at both ends (25, 35).

2. Device according to Claim 1, **characterized by** a common synchronized drive (40) for both shafts (21, 22, 31, 32).

3. Device according to Claim 2, **characterized by** a reversing drive (40).

4. Device according to at least one of Claims 1 to 3, **characterized in that** an upwardly open feed hopper (10) is arranged in the shredding and mixing screw part (21, 31) as a means of supplying solids.

5. Device according to at least one of Claims 1 to 4, **characterized in that** the feeding of liquid (50) is provided in the shredding and mixing screw part (21, 31).

6. Device according to at least one of Claims 1 to 5, **characterized in that** the rotary piston pump (24, 34) has an outlet which is directed upward.

7. Device according to Claim 6, **characterized in that** the rotary piston pump has a means of separating out stones (42) below the pumping region (24, 34).

## Revendications

1. Dispositif pour déchiqueter des matières solides, mélanger avec un fluide et transporter la suspension obtenue dans une installation de transformation ; en particulier une suspension de biosubstrat dans du lisier dans une installation de production de biogaz, avec des équipements d'alimentation pour les matières solides (10) et le fluide (50), un agencement à vis (21, 22, 31, 32) et un équipement de pompe (24, 34) pour la distribution de la suspension, **caractérisé en ce que** l'agencement à vis est un agence à vis double (21, 22, 31, 32) avec deux arbres entraînés, parallèles et agencés l'un à côté de l'autre, sur chacun desquels il est agencé une partie à vis de transport, de déchiquetage et de mélange et un piston rotatif (24, 34) d'une pompe à piston rotatif, et **en ce que** des paliers des arbres et des vis sont prévues aux deux extrémités (25, 35).

2. Dispositif selon la revendication 1, **caractérisé par** un entraînement (40) synchronisé commun pour les deux arbres (21, 22, 31, 32).

3. Dispositif selon la revendication 2, **caractérisé par** un entraînement (40) à marche réversible.

4. Dispositif selon au moins l'une des revendications 1 à 3, **caractérisé en ce que** un entonnoir d'alimentation (10) ouvert vers le haut est agencé dans la partie à vis de déchiquetage et de mélange (21, 31), en tant qu'équipement d'alimentation en matières solides.

5. Dispositif selon au moins l'une des revendications 1 à 4, **caractérisé en ce que** l'alimentation en fluide (50) est prévue dans la partie de déchiquetage et de mélange (21, 31).

6. Dispositif selon au moins l'une des revendications 1 à 5, **caractérisé en ce que** la pompe à piston rotatif (24, 34) comporte une sortie orientée vers le haut.

7. Dispositif selon la revendication 6, **caractérisé en ce que** la pompe à piston rotatif comporte sous la partie de pompage (24, 34) un séparateur de pierres (42).
